(19) **Europäisches Patentamt European Patent Office Office européen des brevets**

(11) **EP 4 699 541 A1**

(12) **EUROPEAN PATENT APPLICATION**

(43) Date of publication:
25.02.2026 Bulletin 2026/09

(21) Application number: 25191967.6

(22) Date of filing: 25.07.2025

(51) International Patent Classification (IPC):
*A61B 6/12* (2006.01)    *A61B 6/00* (2024.01)

(52) Cooperative Patent Classification (CPC):
**A61B 6/12; A61B 6/5264;** A61B 6/487

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **21.08.2024 JP 2024140084**

(71) Applicant: **FUJIFILM Corporation Tokyo 106-8620 (JP)**

(72) Inventor: **Matsuzaki, KAZUKI Tokyo, 106-8620 (JP)**

(74) Representative: **Dehns Germany Partnerschaft mbB Theresienstraße 6-8 80333 München (DE)**

(54) **X-RAY IMAGING APPARATUS AND DEVICE POSITION DETECTION METHOD USING X-RAY IMAGE**

(57) Provided is a technique capable of reducing an influence of aperiodic motion that has occurred during interventional imaging and of monitoring a three-dimensional position of a device with high accuracy.

In order to monitor a device position in interventional imaging, a combination of X-ray images with a minimum influence of body motion is obtained from a plurality of X-ray images acquired at different imaging positions, and the device position is calculated. In this case, movements of feature points extracted from the plurality of X-ray images are analyzed to classify a movement of body motion that has occurred during imaging, a combination of X-ray images to be used for calculating the device position is selected based on classification results, and the device position is calculated.

**FIG. 4**

EP 4 699 541 A1

**Description**

**CROSS-REFERENCE TO RELATED APPLICATIONS**

The present application claims priority under 35 U.S.C. § 119 to Japanese Patent Application No. 2024-140084, filed August 21, 2024. Each of the above application(s) is hereby expressly incorporated by reference, in its entirety, into the present application.

**BACKGROUND OF THE INVENTION**

1. Field of the Invention

The present invention relates to an X-ray imaging apparatus, and more particularly, to a technique for identifying a three-dimensional position of a treatment device inserted into an examination target in an interventional procedure of performing a treatment while checking an image acquired by imaging a treatment target site during a treatment such as a surgery.

2. Description of the Related Art

An X-ray imaging apparatus can identify characteristic structures of a subject in real time and is widely used as imaging means (interventional imaging means) during a treatment such as surgery. In imaging during a treatment, it is extremely important to identify a position of the device with respect to the subject. In the X-ray imaging apparatus, the position of the device within a projection plane, as depicted in an X-ray image, can be identified in real time, but it is difficult to identify the position or structure in a projection direction.

In cone beam CT that acquires a three-dimensional image by rotating an X-ray source and a detector around the subject, the three-dimensional position of the device can be identified from the three-dimensional image. However, the cone beam CT requires more time to acquire a single image as compared to an X-ray imaging apparatus and has a problem in that positional accuracy of the device decreases in a case where body motion or the like of the subject occurs during rotation.

To address this, techniques have been proposed in which an X-ray imaging apparatus configured to allow oscillation of an X-ray source and an X-ray detector is used to acquire X-ray images from different perspectives, and the X-ray images are used to calculate a depth of a device based on a positional relationship of the device in these X-ray images (JP5587861B), and to calculate a three-dimensional position of the device (JP2022-91427A).

**SUMMARY OF THE INVENTION**

In the methods described in the related art in which a plurality of images are used to calculate a position of a target such as a device, imaging times of the plurality of images are different. Therefore, in a case where the body motion of the subject occurs during the acquisition of a plurality of X-ray images, there is a problem in that accuracy of the position calculation deteriorates. In the technique described in JP2022-91427A, in order to reduce an influence of body motion, a parameter serving as an index of a degree of influence of body motion is calculated among combinations of the plurality of images, and the three-dimensional position is calculated by using a combination with a minimum degree of influence of body motion, thereby preventing deterioration in accuracy. In this technique, since a combination of images having approximately the same phase of body motion can be used for periodic motion such as respiratory motion, a highly accurate three-dimensional position can be calculated.

However, in addition to the periodic motion such as respiratory motion, an unexpected movement such as physiological reflexes of the subject (hereinafter, referred to as aperiodic motion) may occur. In a case where such aperiodic motion occurs during imaging, particularly while monitoring the device position, it may be difficult to maintain the accuracy of position calculation even with the technique described in JP2022-91427A.

An object of the present invention is to provide a technique capable of reducing an influence of aperiodic motion that has occurred during imaging and of monitoring a three-dimensional position of a device with high accuracy, thereby providing an X-ray imaging apparatus capable of accurately indicating a device position while performing continuous imaging.

In the present invention, in order to monitor a device position in interventional imaging, a combination of X-ray images with a minimum influence of body motion is obtained from a plurality of X-ray images acquired at different imaging positions, and the device position is calculated. In this case, movements of feature points extracted from the plurality of X-ray images are analyzed to classify a movement of the body motion that has occurred during imaging, a combination of X-ray images to be used for calculating the device position is selected based on classification results, and the device position is calculated.

That is, according to an aspect of the present invention, an X-ray imaging apparatus comprises: an imaging unit including an X-ray source that emits X-rays, and an X-ray detector disposed to face the X-ray source with an examination target interposed therebetween, the imaging unit being configured to generate an X-ray image of the examination target based on X-rays transmitted through the examination target and detected by the X-ray detector; and a processor (calculation unit) configured to analyze a plurality of X-ray images captured at different irradiation angles of X-rays with respect to the examination target to calculate a three-dimensional position of a device inserted into the examination target. The processor (calculation unit) is configured to classify a movement of the examination target from a plurality of X-ray images with different imaging positions, select a combination of two or more X-ray images with different irradiation angles from the plurality of X-ray images based on a classified type of body motion, and calculate the three-dimensional position of the device by using the selected combination.

In addition, according to another aspect of the present invention, a device position detection method using an X-ray image is a method of using a plurality of X-ray images captured at different irradiation angles of X-rays to detect a three-dimensional position of a device depicted in the X-ray images, the method comprising: a step of extracting a feature point from each of the plurality of X-ray images and calculating a movement vector of the feature point between the images; a step of classifying a movement of an imaging target that has occurred during acquisition of the plurality of X-ray images, based on the movement vectors; and a step of selecting an image group acquired in a case where no aperiodic movement occurs, from the plurality of X-ray images, based on the classified movement, in which the three-dimensional position of the device is calculated by using the selected image group.

In the present specification, the term "different imaging positions" includes, or is used synonymously with, a case where the irradiation angles of X-rays emitted from the X-ray source toward the subject are different from each other, and in describing the "imaging position", the term "irradiation angle" may be used instead, and vice versa.

According to the aspects of the present invention, by discriminating a type of body motion that has occurred during imaging of a plurality of X-ray images with different irradiation angles, which are acquired for device position detection, based on movement trajectories of a predetermined shape (feature points) depicted in the X-ray images, and adjusting a combination of X-ray images to be used for device position detection according to whether the body motion is periodic motion or aperiodic motion, it is possible to eliminate the influence of body motion and calculate the three-dimensional position of the device with high accuracy even in a case where aperiodic body motion has occurred.

## BRIEF DESCRIPTION OF THE DRAWINGS

Fig. 1 is a diagram showing an overall outline of an X-ray imaging apparatus to which the present invention is applied.
Figs. 2A and 2B are diagrams showing an example of the X-ray imaging apparatus to which the present invention is applied.
Figs. 3A and 3B are diagrams showing another example of the X-ray imaging apparatus to which the present invention is applied.
Fig. 4 is a functional block diagram of a processor of Embodiment 1.
Fig. 5 is a diagram showing a flow of processing by the processor of Embodiment 1.
Fig. 6 is a diagram showing imaging with different irradiation angles.
Fig. 7 is a diagram showing an example of feature points extracted from an X-ray image.
Fig. 8 is a diagram illustrating determination of a type of body motion by a body motion classification unit.
Fig. 9 is a diagram showing categories of the body motion.
Fig. 10 is a diagram showing an image group acquired in one category of body motion (Case 1).
Fig. 11 is a diagram showing an image group acquired in another category of body motion (Case 2).
Fig. 12 is a diagram showing an example of a combination of images for parameter calculation.
Fig. 13 is a diagram illustrating a parameter calculation method.
Fig. 14 is a functional block diagram of a calculation unit of Embodiment 2.
Fig. 15 is a diagram showing a flow of processing by a processor of Embodiment 2.
Fig. 16 is a diagram illustrating position correction of Embodiment 2.

## DESCRIPTION OF THE PREFERRED EMBODIMENTS

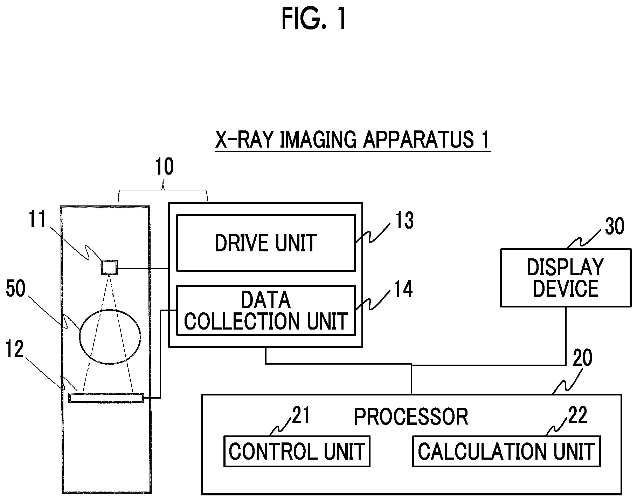

Hereinafter, embodiments of an X-ray imaging apparatus according to the present invention will be described.
As shown in Fig. 1, an X-ray imaging apparatus 1 of the present embodiment comprises an imaging unit 10 comprising an X-ray source 11 and an X-ray detector 12, a processor 20 functioning as a control unit 21 that performs control of the entire apparatus including the imaging unit 10 and a calculation unit 22 that performs processing of image data generated by using X-rays detected by the X-ray detector 12, and a display device 30 that displays an X-ray image and the like. Although not shown, an input device that is used for a user to input necessary commands and data to the control unit 21 and the calculation unit 22, a storage device that stores data necessary for processing, a previously acquired three-dimensional image or the like of a subject 50, and the like may be further provided.

As the X-ray source 11, an X-ray tube is typically used, and the X-ray tube is connected to a high-voltage generating device (not shown). Additionally, the X-ray detector 12 is not limited, but, for example, a flat panel detector (FPD) is used.

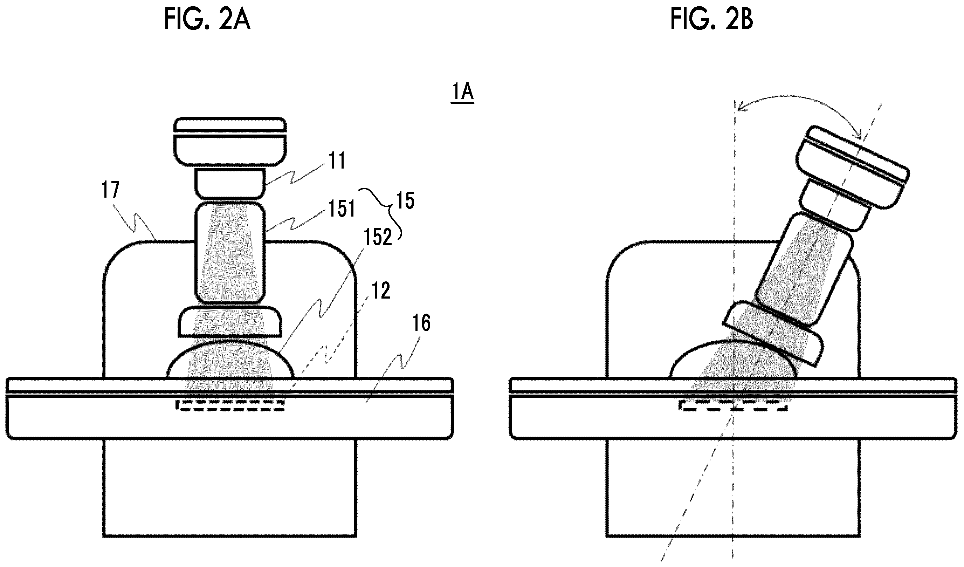

The imaging unit 10 further comprises a drive unit 13 that drives the X-ray source 11, and a data collection unit 14 that receives an electrical signal corresponding to the transmitted X-rays, which is output from the X-ray detector 12, and that collects the electrical signal as two-dimensional image data for each imaging time. The drive unit 13 includes a drive source such as a motor that drives a mechanism (for example, a support mechanism 15 in Figs. 2A and 2B) that supports the X-ray source 11 and the X-ray detector 12 mentioned above, a power supply unit for driving the X-ray source 11, and the like. The image data collected by the data collection unit 14 is displayed on the display device 30 as an X-ray image and is used for processing such as position detection by the calculation unit 22 as necessary.

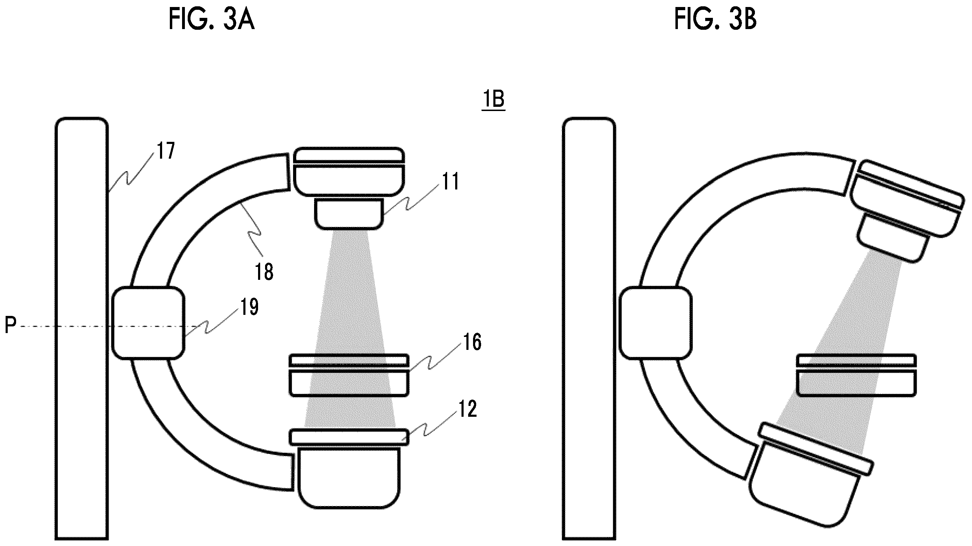

As the X-ray imaging apparatus 1, there are various types of apparatuses depending on a support structure of the X-ray source 11 and the X-ray detector 12, a position of the X-ray source 11, and the like. The present embodiment can be applied to any type of apparatus as long as it has a structure suitable for an interventional procedure and is capable of changing the position of the X-ray source 11 (an irradiation angle of X-rays) with respect to the subject. For example, the present invention can be applied to an X-ray imaging apparatus 1A called an over-tube type fluoroscopic apparatus shown in Figs. 2A and 2B or a C-arm type X-ray imaging apparatus 1B shown in Figs. 3A and 3B.

In the X-ray imaging apparatus 1A shown in Figs. 2A and 2B, the X-ray source 11 is set above a bed 16 on which the subject is laid, and a detector panel constituting the X-ray detector 12 is installed inside the bed 16. In the X-ray imaging apparatus 1A, the X-ray source 11 is fixed to a support stand 17 via the support mechanism 15, and the support mechanism 15 comprises a column 151 that supports the X-ray source 11 and a support arm 152 that rotatably supports the column 151 with respect to the support stand 17. The bed 16 in which the X-ray detector 12 is housed is supported by the support arm 152 to be movable in a horizontal direction and a vertical direction.

In the X-ray imaging apparatus 1A having such a configuration, by rotating the column 151 with respect to the support stand 17, the position of the X-ray source 11 can be changed from the vertical position shown in Fig. 2A to the position shown in Fig. 2B, and the irradiation angle of X-rays with respect to the subject lying on the bed 16 can be changed. In addition, although not shown, a mechanism that moves the column 151 in a direction orthogonal to the paper plane of Figs. 2A and 2B or that rotates the X-ray source 11 (X-ray tube) fixed to the column 151 may be provided, and in this case, the irradiation angle of X-rays can be changed not only two-dimensionally but also three-dimensionally. In the present specification, the term "different irradiation angles" means irradiation angles that are varied two-dimensionally and three-dimensionally.

Figs. 2A and 2B show an over-tube type fluoroscopic apparatus having a configuration in which X-rays are emitted from above the subject, but the same can also be applied to an under-tube type fluoroscopic apparatus in which the X-ray source is disposed below the bed.

Figs. 3A and 3B show the X-ray imaging apparatus 1B having a structure in which the X-ray source 11 and the X-ray detector 12 are supported by a C-arm 18, and the bed 16 on which the subject is laid is disposed in a space between the X-ray source 11 and the X-ray detector 12. The C-arm 18 is fixed to the support stand 17 via a support arm 19, and the position of the C-arm 18 supported by the support arm 19 can be changed, thereby allowing the X-ray source 11 to change from a position directly above the bed 16, as shown in Fig. 3A, to an inclined position, as shown in Fig. 3B, and changing the irradiation angle of X-rays. Additionally, the support arm 19 can be rotated around an axis P with respect to the support stand 17, thereby allowing the X-ray source 11 and the X-ray detector 12 to rotate within a plane orthogonal to the paper plane and changing the irradiation angle of X-rays.

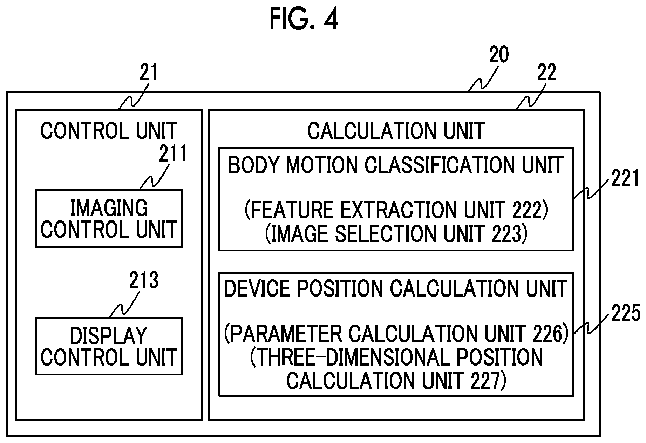

The processor 20 includes the control unit 21 and the calculation unit 22, as shown in Fig. 4. The control unit 21 includes an imaging control unit 211 that controls an operation of the imaging unit 10 to control the movement of the X-ray source 11 by the drive unit 13 and X-ray irradiation from the X-ray source 11, and a display control unit 213 that controls display of the display device 30. For example, the imaging control unit 211 controls the imaging unit 10 to collect a plurality of X-ray images with different imaging times and imaging positions in order to detect a position of a device inserted into the subject during X-ray imaging.

The calculation unit 22 comprises a body motion classification unit 221 that analyzes image data collected by the data collection unit 14 for a certain period of time, that is, the plurality of X-ray images, and that classifies the type of body motion of the subject, and a device position calculation unit 225 that calculates the device position based on the classification of the body motion by the body motion classification unit 221. In the embodiment shown in Fig. 4, the body motion classification unit 221 extracts feature points included in the X-ray image (feature extraction unit 222) in order to detect the body motion from the X-ray image and classifies the body motion from movements of the feature points in the plurality of X-ray images with different imaging positions. For example, the device position calculation unit 225 selects a plurality of combinations from the plurality of X-ray images, calculates a parameter serving as an index of a degree of influence of body motion for each combination (parameter calculation unit 226), decides on a combination for calculating the device position based on the calculated parameter, and calculates the three-dimensional position of the device by using the decided-on combination (three-dimensional position calculation unit 227).

In Fig. 1, the processor 20 is shown as a single block, but in the present specification, the processor comprises, for example, a CPU or a GPU, and a memory and includes various computers that implement functions by software, and hardware such as an application-specific integrated circuit (ASIC), a field programmable gate array (FPGA), and a programmable IC, and the functions of each unit included in the processor mentioned above can be implemented by one or a plurality of processors. For example, the control unit 21 and the calculation unit 22 can implement some or all of the functions of the respective subunits included in the control unit 21 and the calculation unit 22 by the above-described software or hardware alone, or a combination thereof.

The X-ray imaging apparatus of the present embodiment has a feature in the processing of the processor 20 that detects the position of the device inserted into the examination target by using the X-ray imaging apparatus as the interventional imaging means, and the detection of the device position is based on a technique of detecting the three-dimensional position of the device by using a plurality of X-ray images acquired by the imaging unit 10 at different imaging positions. Then, the body motion of the subject that has occurred during the acquisition of the plurality of X-ray images is analyzed, the body motion is classified, a combination of images to be used for calculating the three-dimensional position is decided on from among the plurality of X-ray images based on the type of body motion, a combination with a smallest influence of body motion is selected from a plurality of combinations of images, and the three-dimensional position is calculated. Specific methods of classifying the body motion and calculating the three-dimensional position will be described in detail in the following embodiments.

With the X-ray imaging apparatus of the present embodiment, by classifying the body motion prior to the calculation of the device position, and particularly, by specifying the range of images to be used for calculating the device position based on whether the body motion is periodic motion or aperiodic motion, it is possible to prevent a decrease in the accuracy of the three-dimensional position calculation in a case where the body motion, particularly aperiodic motion, occurs, thereby enabling highly accurate position detection.

Embodiment 1

In the present embodiment, the three-dimensional position of the device is detected by using, as the plurality of X-ray images, X-ray images acquired by the imaging unit 10 respectively at a plurality of irradiation angles within a predetermined angle range.

In addition, in the present embodiment, in order to classify the movement of the subject while the X-ray images are being acquired, the calculation unit 22 extracts the feature points of the device or the subject on the X-ray image from each of a plurality of X-ray images with different irradiation angles and determines whether the movement of the subject including the vicinity of the device is a periodic movement or an aperiodic movement based on a change in the feature point position. A plurality of X-ray images (image group) to be used for calculating the three-dimensional position are decided on based on the determination result. As shown in Fig. 4, the body motion classification unit 221 may include an image selection unit 223 as a functional unit that selects an image group. The image selection unit 223 is used for the subsequent device position calculation process, that is, parameter calculation for calculating the three-dimensional position, based on a predetermined criterion, in a case where the plurality of images are divided into one or more image groups based on the categories of body motion.

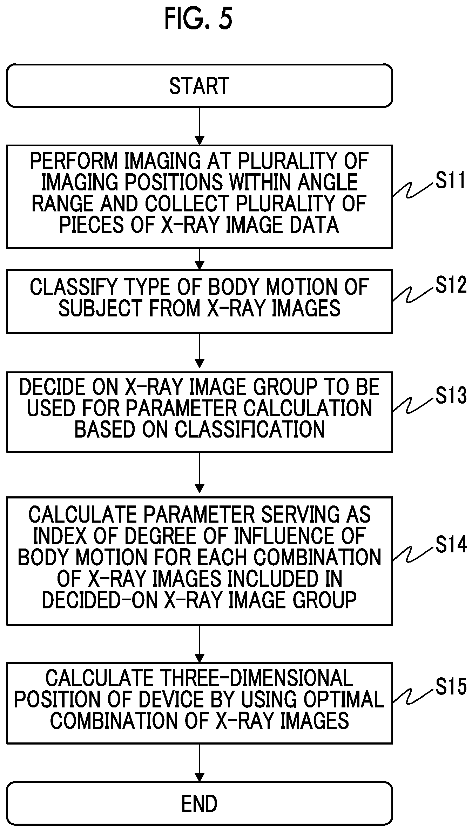

Hereinafter, an operation of the X-ray imaging apparatus of the present embodiment, mainly an operation of the processor, will be described with reference to a flow shown in Fig. 5.

Imaging Step: S11

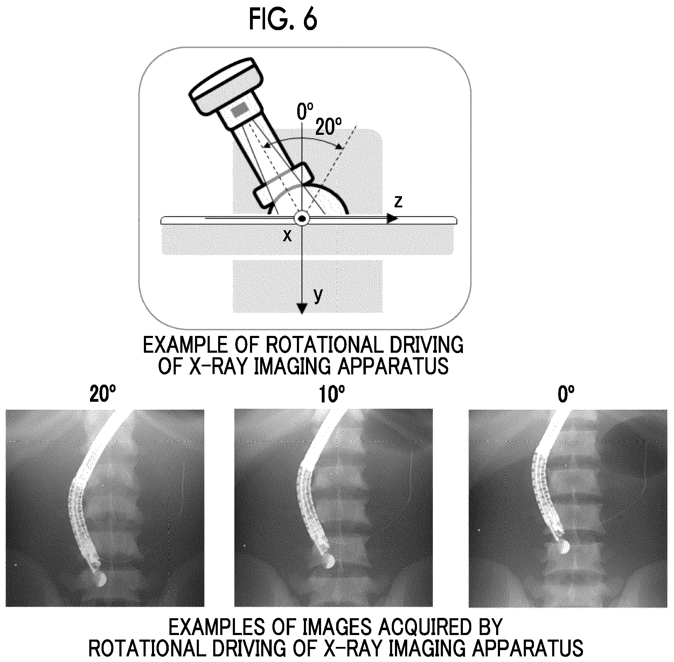

The imaging unit 10 starts imaging and acquires X-ray images at a plurality of irradiation angles while rotating the X-ray source 11 and the X-ray detector 12 within an angle range set in advance. The predetermined angle is not particularly limited as long as it falls within the movable range of the X-ray imaging apparatus. For example, in a case where the initial positions of the X-ray source 11 and the X-ray detector 12 are set to zero degrees, an angle range of $\pm 10$ degrees (angle range of 20 degrees) may be used. Fig. 6 shows an example in which X-ray images are acquired in an angle range of 20 degrees. Imaging may be performed only once at each of a plurality of irradiation angles within the set angle range, or imaging may be repeatedly performed a plurality of times at a predetermined irradiation angle and a plurality of X-ray images may be acquired for each irradiation angle. In a case where a plurality of X-ray images are acquired for each irradiation angle, imaging within a predetermined angle range may be repeatedly performed a plurality of times, or imaging may be performed a plurality of times for each irradiation angle. Additionally, the imaging method may be step-by-step, or in a case of the X-ray source 11 or the C-arm type X-ray imaging apparatus, imaging may be continuously performed while the X-ray source 11 and the X-ray detector 12 are continuously rotated. In the latter case, a plurality of X-ray images are obtained at the same irradiation angle by repeatedly performing imaging within the predetermined angle range.

The imaging target is a region including an examination site of the subject, and in the present embodiment, imaging is targeted for a case where a device such as a catheter provided with a guide wire is inserted toward the examination site and an interventional procedure is performed. The X-ray image captures the examination site and a part of the advancing device.

Body Motion Classification Step: S12

The calculation unit 22 determines the presence or absence of the movement of the subject and classifies the type of movement by using the plurality of X-ray images obtained by the above-described imaging. Therefore, first, the feature extraction unit 222 extracts the feature points of the device or a predetermined site of the subject from each X-ray image. The feature points are feature points of a shape of a tissue or an object that can be detected in the X-ray image, and are not particularly limited as long as they correspond to portions in which significant changes in brightness are observed on the X-ray image. The feature points may be either the feature points of the tissue of the subject or the feature points of the device, but in order to determine the body motion related to the device, it is suitable to track the feature points of the device.

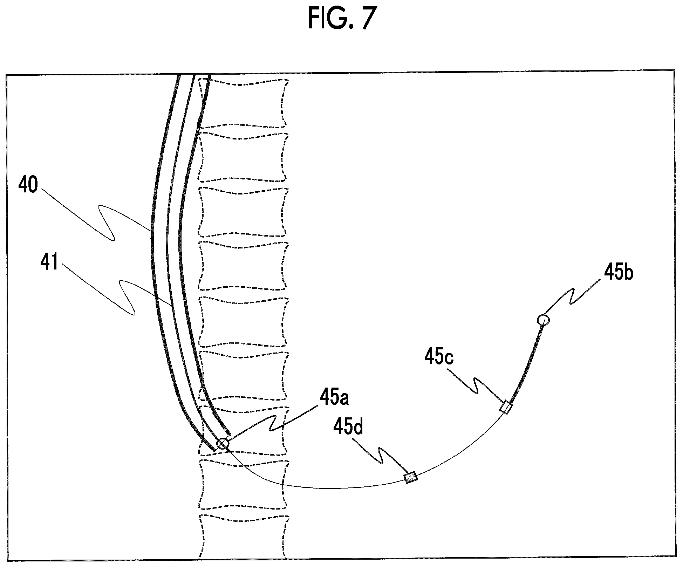

Fig. 7 shows an example of the feature points in a case where the device is an endoscope 40 provided with a guide wire 41. As shown in Fig. 7, as the feature points, an end portion 45a of the guide wire 41 on an endoscope 40 side, a distal end 45b in a direction of advancement, a boundary point 45c between a transmissive part and an impermeable part of the guide wire 41, a marker 45d (a marker made of an X-ray impermeable material) provided on the catheter, and the like can be detected. The feature extraction unit 222 extracts points where such brightness changes are characteristic by using general image processing techniques and specifies position coordinates of the feature points in the image. The feature points are used for analysis of body motion and calculation of the three-dimensional position of the device, which will be described below. The number of feature points to be extracted may be one or a plurality of feature points. However, in calculating the three-dimensional position of the device, by using the positions of a plurality of feature points, information for identifying the position of the device having a certain length or size can be obtained.

The body motion classification unit 221 obtains a change in the position of the feature point based on the coordinates of the feature point in each X-ray image and classifies the body motion of the subject. As the change in the position of the feature point, specifically, a movement distance or a vector of the feature point between adjacent images, a trajectory (movement vector) of the movement of the feature point from a first acquired image to a last acquired image among the plurality of images, an average of the movement vectors, a sum of the movement vectors, and the like are calculated. The body motion is classified into, for example, periodic motion or aperiodic motion based on the calculated change in the position of the feature point. As a criterion for classification, a combination of the movement vector, the average of the movement vectors, and the sum of the movement vectors may be used, or any one of them may be used alone. In either case, a predetermined threshold value is set, and periodic motion and aperiodic motion are determined and classified based on whether the temporal displacement of the feature point falls within or exceeds the threshold value.

The classification may include not only two types, periodic motion and aperiodic motion, but may also be more finely classified, such as: no body motion; periodic motion (movement within a predetermined range); aperiodic motion 1 (movement involving a large positional change that exceeds the predetermined range but returns to the original position); and aperiodic motion 2 (movement involving a large positional change without returning to the original position).

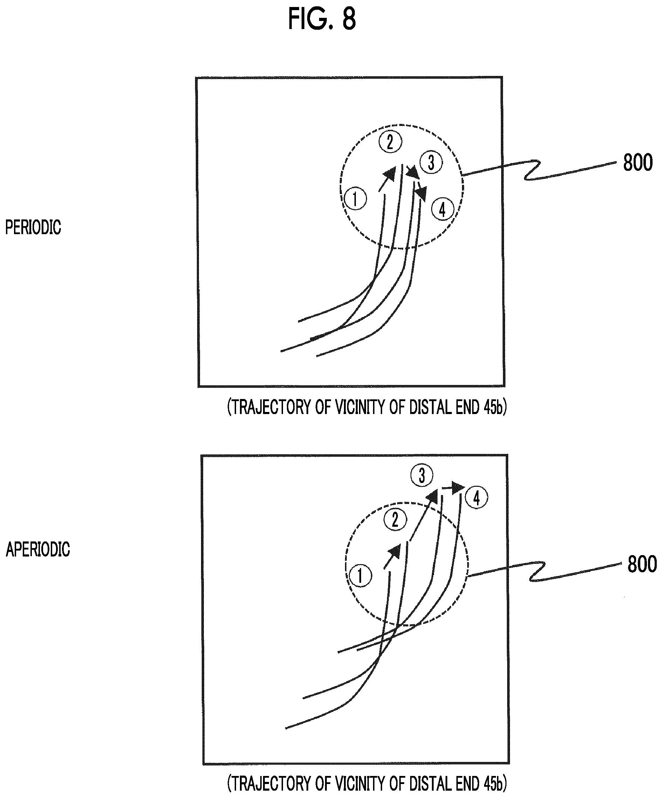

Fig. 8 shows an example in which the body motion is classified into periodic motion and aperiodic motion. This example is an example in which the distal end 45b of the guide wire 41 is tracked as a feature point. The feature point is shown to have moved from position 1 to position 4 indicated by circled numbers, and classification is performed into periodic motion or aperiodic motion based on the movement vectors of the feature point or the average thereof. In a case where classification is based on the movement vectors, for example, the body motion is determined to be periodic motion in a case where the average of the movement vectors falls within a range (predetermined threshold value range) 800 of the periodic motion indicated by a dotted line, and the body motion is determined to be aperiodic motion in a case where the average falls outside the range 800.

The period for obtaining the vectors (the period for obtaining vectors of four points in Fig. 8) can be, for example, as short as the interval between acquisition of projection images. The period for determining the body motion based on the obtained vectors depends on the type of body motion. For example, the period can be set to once per second in a case of short-period body motion such as pulsation, and the period can be set to once every three to five seconds in a case of respiratory motion.

In addition, in a case where the body motion is respiratory motion or pulsation, and the range of the movements (amount of displacement) of surrounding tissues caused by pulsation or respiratory motion is known, the range 800 for periodic motion can be set based on the amount of displacement. For example, the amount of displacement is about 10 mm in a case of pulsation and about 20 mm in a case of respiratory motion. In a case of periodic motion, the average of the movement vectors of the feature point converges to approximately zero over time. However, in a case of aperiodic motion, the average of the movement vectors of the feature point does not converge to zero and is a large value. Therefore, in a case where the average or sum of the movement vectors approaches zero, it is determined to be periodic motion, and in a case where the average or sum of the movement vectors exceeds a threshold value (TH), it is determined to be aperiodic motion.

<Decision of X-ray Image Group: S13>

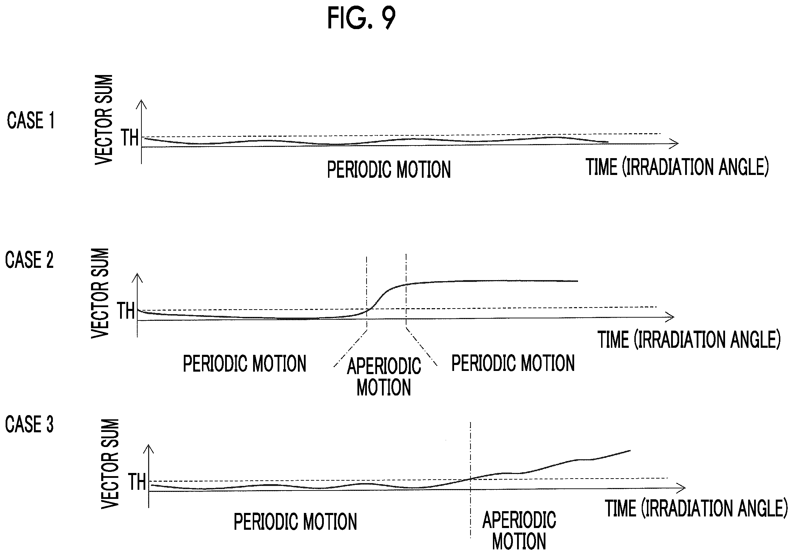

The body motion classification unit 221 (image selection unit 223) specifies an image group to be used for three-dimensional position detection based on classification results (the category of body motion) of the body motion that has occurred during the acquisition of the plurality of images, among the plurality of images acquired within a predetermined angle range. There are various categories of body motion that may occur while a plurality of images are being acquired. For example, as shown in Fig. 9, there are possible cases such as: a case where all X-ray images are acquired in a state in which body motion is classified as no body motion or periodic motion (Case 1); a case where imaging starts in a state in which body motion is classified as no body motion or periodic motion, but aperiodic motion occurs at a certain point in time, followed by a return to periodic motion at the displaced position (Case 2); and a case where periodic motion is followed by aperiodic motion that continues for a certain period of time (Case 3). The vertical axis of Fig. 9 indicates the magnitude of the average or sum of the movement vectors, but may indicate the amount of displacement of the body motion. "TH" is the threshold value.

The image selection unit 223 specifies the image group to be used for calculating the three-dimensional position based on the classification of the body motion, the classification of the X-ray image based on the classification of the body motion, and the category corresponding to the occurrence of the body motion. As a criterion for specifying the image group, the number of images belonging to the image group, a priority of the image group, and the like can be used. In a case where there are a plurality of criteria, a priority of applying the criteria may be set in advance.

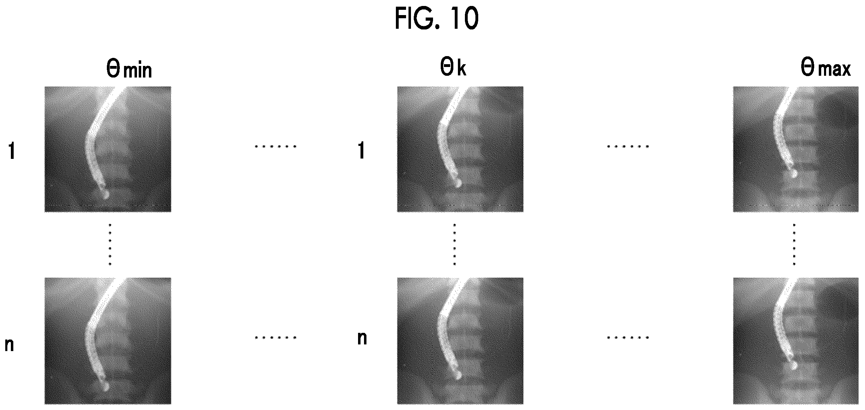

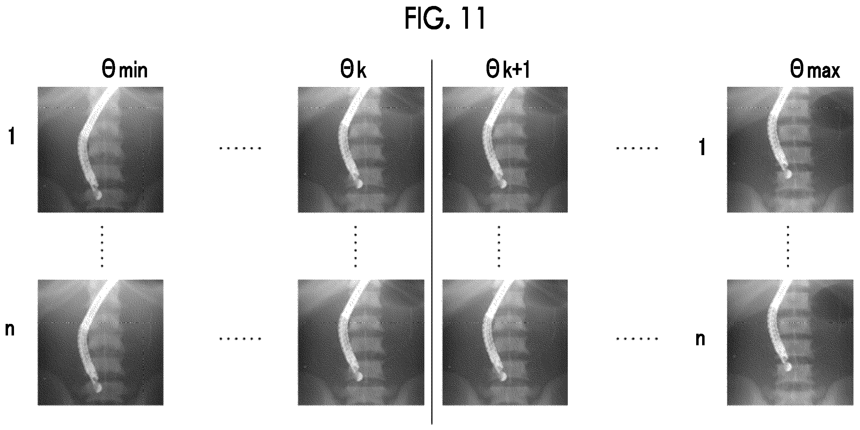

Figs. 10 and 11 show image examples obtained by different categories of body motion. Fig. 10 is an example of Case 1 mentioned above, where there is no body motion within the predetermined angle range or the displacement range of the device is limited to periodic motion. In this case, all the X-ray images obtained within the predetermined angle range are used for device position calculation.

Fig. 11 is an example of Case 2 mentioned above, where within the irradiation angle range ($\Theta$min to $\Theta$max), body motion is periodic motion up to an irradiation angle $\Theta$k of X-rays. However, body motion classified as aperiodic motion occurs between the irradiation angle $\Theta$k and an irradiation angle $\Theta$k+1, or within a predetermined range encompassing the irradiation angle Ok and the irradiation angle $\Theta$k+1. After that, the device position that has shifted from its initial position returns to periodic motion. Here, in accordance with the classification of the body motion, the plurality of images are divided into an image group of irradiation angles ($\Theta$min to $\Theta$k-1), an image group of irradiation angles ($\Theta$k to $\Theta$k+1) acquired during aperiodic motion, and an image group ($\Theta$k+2 to $\Theta$max) after the aperiodic motion. In this case, for example, in Case 2 mentioned above, in a case where the plurality of acquired X-ray images include a first image group and a second image group, an image group having a greater number of belonging images is used as the image group to be used for calculating the three-dimensional position. Alternatively, in a case where the number of images belonging to the first image group is equal to or greater than a predetermined number, the first image group is specified, and in a case where the number of images belonging to the first image group is less than the predetermined number, the second image group is used. Alternatively, an image group having a larger angular width between $\Theta$min to $\Theta$k-1 and $\Theta$k+2 to $\Theta$max may be used as the image group to be used for calculating the three-dimensional position.

Although not shown, in a case of Case 3 shown in Fig. 9, the image group acquired during periodic motion is selected. Additionally, in a case where a sufficient number of images for ensuring the accuracy of the three-dimensional position detection cannot be obtained, such as in a case where the number of images included in the image group acquired during periodic motion is small, the acquired image data may be discarded, and imaging within the predetermined angle range may be performed again. That is, the classification result is passed from the body motion classification unit 221 to the control unit 21, and the imaging control unit 211 controls the imaging unit 10 to perform re-imaging.

Device Position Detection

Parameter Calculation: S14

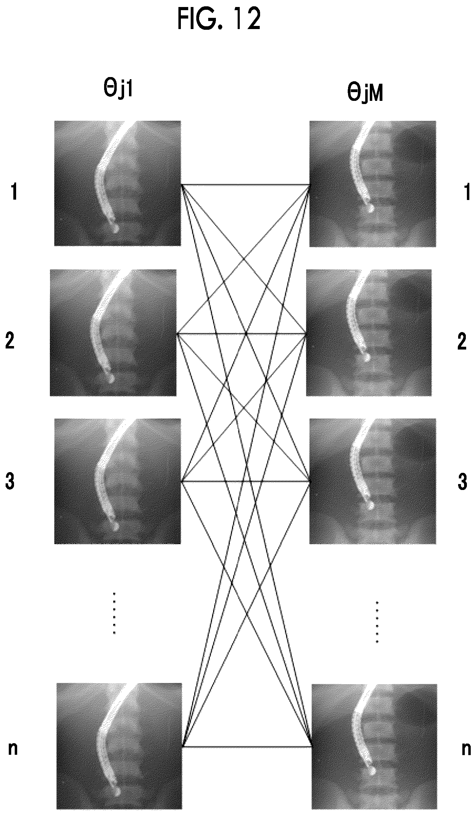

The parameter calculation unit 226 uses the plurality of X-ray images belonging to the specified image group to calculate a parameter serving as an index of the influence of body motion in a plurality of combinations of X-ray images with different imaging positions. The combination of X-ray images is not limited, but, for example, as shown in Fig. 12, in a case where a plurality (N) of X-ray images are obtained at a plurality (M) of imaging positions (angle ranges ($\Theta$j1 to OjM)), the X-ray images from 1 to N at each irradiation angle are combined in an all-to-all manner with X-ray images at different irradiation angles to form M $\times$ N combinations.

As the parameter, a distance (distance between two straight lines) between straight lines determined by the position of the X-ray source 11 and the position of the feature point on the image in a case where each X-ray image is acquired can be used. In two X-ray images to be compared, in a case where no body motion occurs and the feature point position does not change, the two straight lines intersect each other, and the feature point is obtained as a single coordinate. However, in a case where the position of the feature point is displaced due to body motion, the two straight lines do not intersect each other, and the distance between the two straight lines changes depending on the magnitude of the displacement. That is, the magnitude of the influence of body motion is reflected in the distance between the two straight lines.

In addition to the distance between two straight lines, as the parameter, other parameters can also be used such as a perimeter length or an area of a polygon generated by connecting midpoints of line segments represented as the distance between two straight lines using three or more X-ray images as disclosed in JP2022-91427A. In the following description, for simplicity of the description, a case will be described where the distance between two straight lines is calculated.

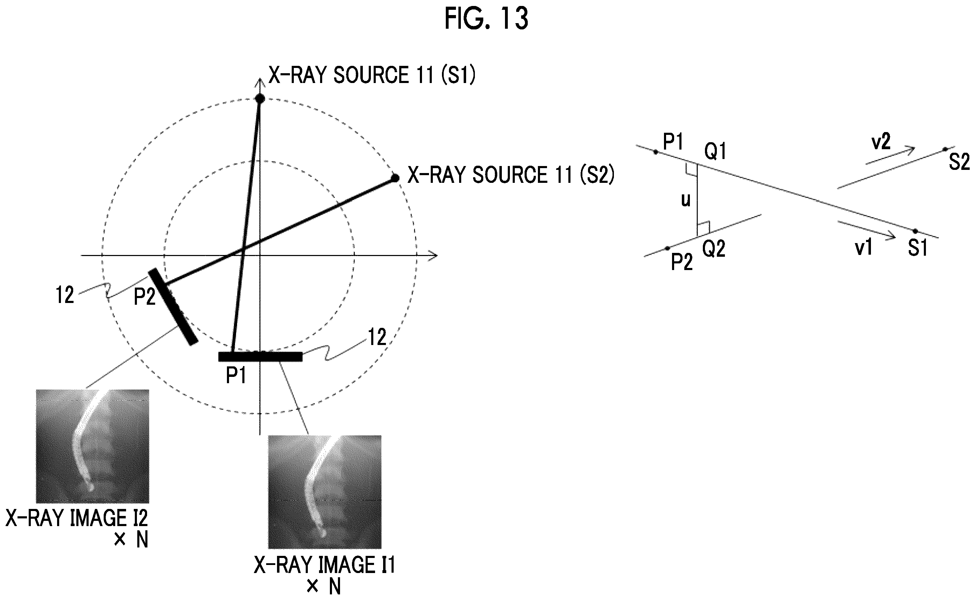

The parameter calculation unit 226 calculates the distance between two straight lines as follows. As shown in Fig. 13, a position of the X-ray source 11 at a first imaging position is denoted by S1, a position thereof at a second imaging position is denoted by S2, and positions of the device on the X-ray detector 12 at the first imaging position and the second imaging position are denoted by P1 and P2, respectively. The positions P1 and P2 of the device on the X-ray detector 12 can be calculated from the geometric disposition of the X-ray source 11 and the X-ray detector 12 and the positions of the device on an X-ray image I1 and an X-ray image I2 acquired at respective positions. Here, in a case where a straight line connecting S1 and P1 is represented by a vector v1 and a straight line connecting S2 and P2 is represented by a vector v2, a line segment Q1-Q2 (vector u) located at the shortest distance between the two straight lines can be obtained by Equation 1.

$$[\text{Equation 1}]$$

$$Q1 = P1 + (D1 - D2 * Dv) / (1 - Dv * Dv) * v1$$

$$Q2 = P2 + (D2 - D1 * Dv) / (Dv * Dv - 1) * v2$$

where

$$D1 = (P2 - P1) \cdot v1$$

$$D2 = (P2 - P1) \cdot v2$$

$$Dv = v1 \cdot v2$$

("*" denotes a vector inner product, and "·" denotes a scalar product or a scalar-vector product)

The parameter calculation unit 226 calculates a length D of the line segment (distance between Q1 and Q2), that is, the distance between the two straight lines, for the combination of all the X-ray images belonging to the image group selected by the image selection unit 223.

Optimal Combination Selection and Three-Dimensional Position Calculation: S15

After the parameter calculation unit 226 calculates the parameters for all the combinations, the three-dimensional position calculation unit 227 calculates the three-dimensional position of the feature points of the device by using a combination with a minimum parameter among all the combinations. As mentioned above, the combination with the minimum parameter (here, the distance between two straight lines) is a combination of X-ray images in which the influence of body motion is the smallest and the difference in displacement is the smallest. Therefore, the device position can be calculated with high accuracy by using such a combination. In a case where the parameter is the distance between two straight lines, the three-dimensional position of the device can be calculated as the midpoint of the line segment that specifies the distance. That is, the three-dimensional position calculation unit 227 calculates the device position (coordinates of the feature point) by using the specific values Q1 and Q2 (refer to Fig. 13 and Equation 1) of the parameter according to Equation 2.

[Equation 2]

$$Q = (Q1 + Q2) / 2$$

In a case where the parameter is a parameter calculated based on a figure calculated by using three or more X-ray images, a centroid or a center of the figure can be calculated as the feature point position (the device position). In a case where a plurality of feature points are extracted, the position is calculated for each feature point.

The device position calculated by the three-dimensional position calculation unit 227 is, for example, passed to the display control unit 213, and is mapped on the previously acquired three-dimensional image of the subject and displayed on the display device 30.

Steps (S11 to S15) from the imaging within the predetermined angle range to the device position calculation mentioned above are repeated each time the device position changes or at predetermined periodic intervals while the interventional procedure is being performed, and a mapping result is updated and displayed on the display device 30 each time.

According to the present embodiment, body motion is classified based on the trajectories of the device (feature points) on the X-ray images, and X-ray images to be used in the subsequent three-dimensional position detection process including parameter calculation and device position calculation are selected based on the category of body motion that has occurred, thereby enabling highly accurate three-dimensional position detection and enhancing the effectiveness of the X-ray imaging apparatus as interventional imaging means. In particular, it is possible to avoid the influence of aperiodic body motion that occurs in a case where a plurality of X-ray images are acquired at different imaging positions, that is, at different times, for the position detection, thereby improving the accuracy of position detection.

In addition, according to the present embodiment, in a case where the three-dimensional position is obtained by calculating a parameter from a combination of a plurality of X-ray images, image data affected by aperiodic body motion can be excluded from the calculation, thereby reducing the load on the calculation unit caused by parameter calculation.

In the above description, a case has been described where the classification of the body motion and the selection of the image group based on the category are automatically performed by the calculation unit 22, but it is also possible to present the intermediate progress to the user or to allow user intervention in the determination or selection, and such modification examples are also included in the present invention.

Embodiment 2

The present embodiment is an embodiment that addresses changes in the position of the target on the image caused by the irradiation angle, and is characterized by having a function of handling the change in the position of the feature point in the body motion classification.

The present embodiment is the same as in Embodiment 1 in that imaging is performed within a predetermined irradiation angle range of the X-ray source to obtain a plurality of X-ray images, body motion that has occurred during the acquisition of the plurality of X-ray images is classified, and parameters are calculated by using a plurality of combinations of X-ray images with different irradiation angles among the plurality of X-ray images to calculate the three-dimensional position of the device.

Hereinafter, the present embodiment will be described focusing on points different from Embodiment 1. In the following description, the contents common to Embodiment 1 will be referred to with reference to the drawings used in the description of Embodiment 1, and the overlapping description will be omitted.

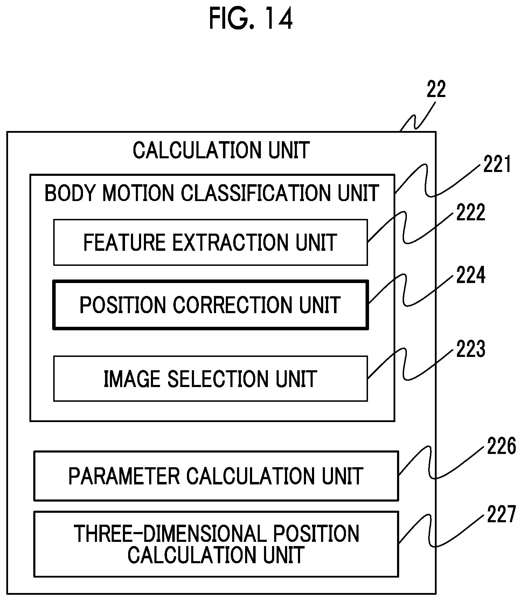

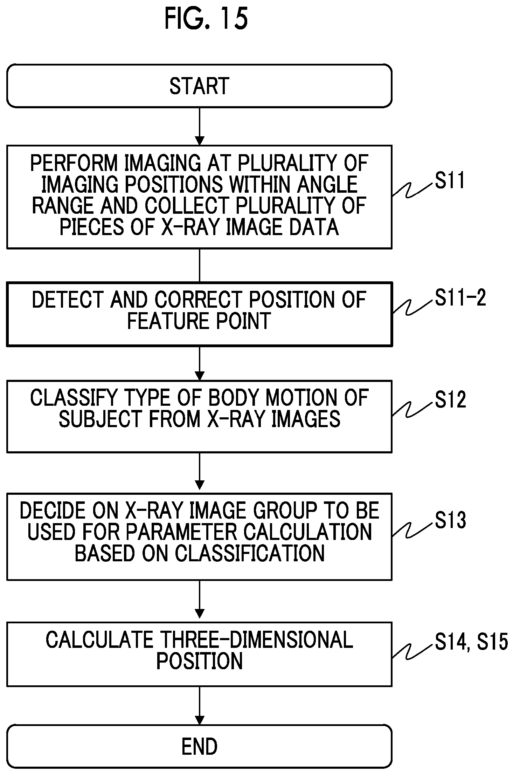

Fig. 14 shows the functions of the calculation unit 22 of the present embodiment, and Fig. 15 shows the flow of processing of the calculation unit 22. As shown in Fig. 14, the body motion classification unit 221 of the present embodiment comprises a position correction unit 224 in addition to the feature extraction unit 222 and the image selection unit 223 in Embodiment 1. As shown in S11-2 of Fig. 15, the position correction unit 224 corrects the coordinates (positions) of the feature points extracted by the feature extraction unit 222 from each X-ray image.

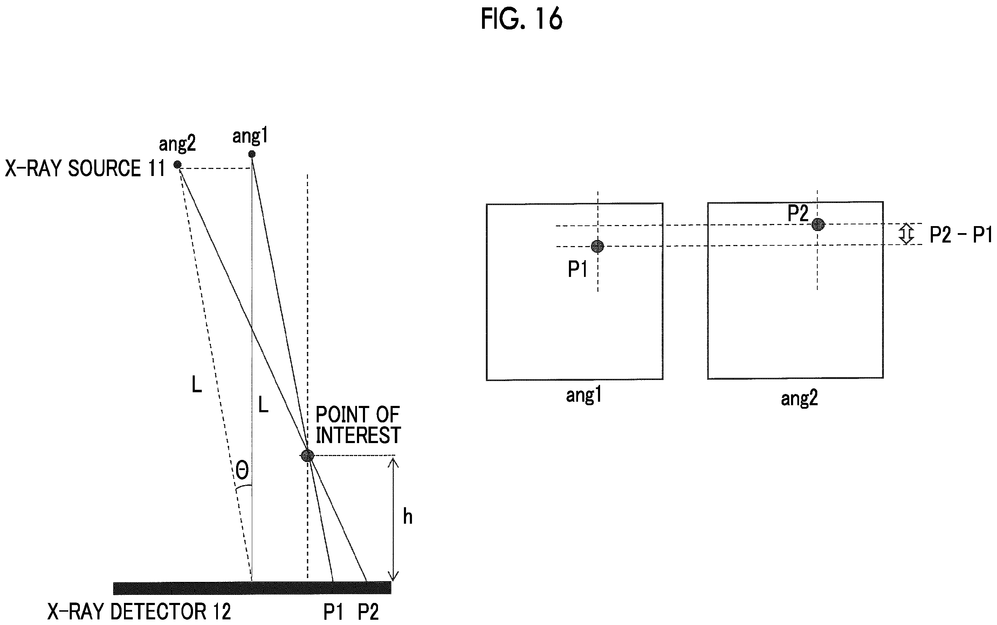

In order to describe the function of the position correction unit 224, a change in the position of a point of interest (for example, a feature point) on the image due to the irradiation angle will be described with reference to Fig. 16.

In the X-ray imaging apparatus of the type in which the X-ray detector 12 is fixed to the bed with the subject placed thereon, as shown in Fig. 16, the position of the target (indicated as a single point of interest in Fig. 16) depicted in the X-ray image between the X-ray source 11 and the X-ray detector 12 is different between a case where the X-ray source 11 is at an irradiation angle ang1 and a case where the X-ray source 11 has moved by an angle Θ from the irradiation angle ang1 to an irradiation angle ang2, as shown on the left side in the drawing. That is, even in a case where the point of interest does not move, changing the irradiation angle causes the point of interest to move on the image, making it appear as if the point of interest has moved. Since the irradiation angle of the X-ray source 11 typically changes within a single plane intersecting the X-ray detector 12, the position of the point of interest moves in one direction (the vertical direction in Fig. 16) on the X-ray image.

Here, in a case where the position of the point of interest at the irradiation angle ang1 is denoted by P1, and the position of the point of interest at the irradiation angle ang2 is denoted by P2, a movement amount (P2-P1) of the point of interest due to the irradiation angle can be represented by Equation 3.

[Equation 3]

$$P2\text{-}P1 = h * L * \sin\Theta / (L * \cos\Theta - h) = \Delta P(\Theta) \qquad (3)$$

In the equation, L represents a distance from the X-ray source 11 to the X-ray detector 12, and h represents a distance from the X-ray detector 12 (for example, an upper surface of the flat panel detector) to the point of interest.

The distance h from the X-ray detector 12 to the point of interest can be obtained, for example, by using known distance from the upper surface of the bed to the X-ray detector 12 and the statistically known distance from the bed surface to a predetermined organ of the subject into which the device is inserted. As a result, it is possible to obtain the displacement for each irradiation angle, that is, a correction amount $\Delta P(\Theta)$ of the displacement, from Equation (3).

The position correction unit 224 corrects coordinates P' of the feature point actually extracted by the feature extraction unit 222 for each irradiation angle by using the correction amount $\Delta P(\Theta)$ to obtain a position P of the feature point for each irradiation angle (P = P' + $\Delta$P or P = P' - $\Delta$P. The sign of $\Delta$P depends on the sign of 8 with respect to the reference irradiation angle).

Subsequent processing is the same as in Embodiment 1, and the body motion classification unit 221 calculates the movement vectors of the feature points within the predetermined angle range by using the positions of the feature points that have been corrected by the position correction unit 224, classifies the body motion (S12), and selects a plurality of X-ray images (image group) to be used for device position calculation based on the classification. The device position calculation unit 225 calculates parameters from various combinations of X-ray images by using the selected image group and calculates the three-dimensional position of the device from a combination of images that yields the parameter with the smallest degree of influence of body motion (S13 to S15).

According to the present embodiment, in addition to the same effects as those in Embodiment 1, it is possible to perform highly accurate body motion classification by correcting the change in the positions of the feature points in the images with different irradiation angles in the X-ray imaging apparatus in which the irradiation angle of X-rays with respect to the X-ray detector changes. As a result, even in a case where not only periodic body motion but also aperiodic body motion occurs, it is possible to detect the three-dimensional position of the device while avoiding the influence of both the periodic and aperiodic body motion.

In addition, according to the present embodiment, since it is possible to acquire images in a plurality of directions with aligned positions while continuously changing the angle, it is possible to acquire the three-dimensional position as quickly and accurately as possible while taking body motion into consideration. That is, in a case where imaging is repeated at each angle by fixing the angle and acquiring data for one period, the accuracy of position detection deteriorates in a case where aperiodic body motion occurs during movement between angles. However, according to the present embodiment, since images are acquired continuously, it is possible to determine whether aperiodic body motion and the like have occurred during the acquisition, thereby improving the temporal resolution of determination and position detection and shortening the acquisition time.

Although the embodiments of the present invention have been described above, embodiments in which known configurations or functions are added to the above-mentioned embodiments, or embodiments in which configurations or functions that can be omitted in the present invention are also included in the present invention.

Explanation of References

1: x-ray imaging apparatus
10: imaging unit
20: processor
21: control unit
22: calculation unit
30: display device
40: endoscope
41: guide wire (device)
50: subject
211: imaging control unit
213: display control unit

**EP 4 699 541 A1**

221: body motion classification unit
222: feature extraction unit
223: image selection unit
224: position correction unit
225: device position calculation unit
226: parameter calculation unit
227: three-dimensional position calculation unit

**Claims**

1. An X-ray imaging apparatus (1) comprising:

an imaging unit (10) including an X-ray source (11) that emits X-rays, and an X-ray detector (12) disposed to face the X-ray source (11) with an examination target interposed therebetween, the imaging unit (10) being configured to generate an X-ray image of the examination target based on X-rays transmitted through the examination target and detected by the X-ray detector (12); and
a processor (20) configured to analyze a plurality of X-ray images captured at different irradiation angles of X-rays with respect to the examination target to calculate a three-dimensional position of a device inserted into or attached to the examination target,
wherein the processor (20) is configured to classify a movement of the examination target from a plurality of X-ray images with different imaging positions, select a combination of two or more X-ray images with different irradiation angles from the plurality of X-ray images based on a classified type of body motion, and calculate the three-dimensional position of the device by using the selected combination.

2. The X-ray imaging apparatus (1) according to claim 1,
wherein the processor (20) is configured to extract feature points from at least one of the examination target or the device from the plurality of X-ray images and classify the body motion based on trajectories of positions of the extracted feature points in the plurality of X-ray images.

3. The X-ray imaging apparatus (1) according to claim 1,
wherein the processor (20) is configured to extract feature points of at least one of the examination target or the device from the plurality of X-ray images and classify the body motion based on movement vectors of the feature points in the plurality of images.

4. The X-ray imaging apparatus (1) according to claim 3,
wherein the processor (20) is configured to determine the movement of the examination target as a periodic movement in a case where the movement vectors or an average of the movement vectors is within a threshold value set in advance, and determine the movement of the examination target as an aperiodic movement in a case where the movement vectors or the average of the movement vectors exceeds the threshold value set in advance.

5. The X-ray imaging apparatus (1) according to claim 4,
wherein the processor (20) is configured to, in a case where the plurality of X-ray images include X-ray images acquired when the movement of the examination target is an aperiodic movement as a result of classifying the body motion, select an image group from the plurality of X-ray images, excluding the X-ray images acquired during the aperiodic movement, and calculate the three-dimensional position of the device by using the selected image group.

6. The X-ray imaging apparatus (1) according to claim 4,
wherein the processor (20) is configured to, in a case where the plurality of X-ray images include a plurality of image groups acquired when there is no movement or a periodic movement before and after an aperiodic movement as a result of classifying the body motion, select an image group including a greater number of images and calculate the three-dimensional position of the device by using the selected image group.

7. The X-ray imaging apparatus (1) according to claim 1,
wherein the processor (20) is configured to calculate a parameter serving as an index of an influence of body motion for each of a plurality of combinations consisting of two or more X-ray images included in the plurality of X-ray images, select a combination of X-ray images with a smallest influence of body motion based on the parameter, and calculate the three-dimensional position of the device.

8. The X-ray imaging apparatus (1) according to claim 1,
   wherein the processor (20) is configured to

   calculate, for each of the plurality of X-ray images, a straight line connecting the X-ray source and a feature point in the X-ray image and calculate a distance between two straight lines from a plurality of the straight lines calculated for each X-ray image, and
   calculate the three-dimensional position of the device by using a combination of X-ray images in which the distance between the two straight lines is shortest, among combinations of X-ray images with different irradiation angles.

9. The X-ray imaging apparatus (1) according to claim 2,

   wherein the X-ray imaging apparatus (1) changes the irradiation angle of X-rays by moving a position of the X-ray source with respect to the examination target and the X-ray detector, and
   the processor (20) includes a position correction unit that corrects the positions of the feature points based on the irradiation angle of the X-ray source (11), and is configured to classify the body motion based on trajectories of the feature points after the position correction.

10. A device position detection method of using a plurality of X-ray images captured at different irradiation angles of X-rays to detect a three-dimensional position of a device depicted in the X-ray images, the method comprising:

   extracting a feature point from each of the plurality of X-ray images and calculating a movement vector of the feature point between the images;
   classifying a movement of an imaging target that has occurred during acquisition of the plurality of X-ray images, based on the movement vectors;
   selecting an image group acquired in a case where no aperiodic movement occurs, from the plurality of X-ray images, based on the classified movement; and
   calculating the three-dimensional position of the device by using the selected image group.

# FIG. 1

## X-RAY IMAGING APPARATUS 1

10

11

50

12

| DRIVE UNIT | 13 |

| DATA COLLECTION UNIT | 14 |

DISPLAY DEVICE 30

20

PROCESSOR

21 | CONTROL UNIT |

22 | CALCULATION UNIT |

FIG. 2A

FIG. 2B

EP 4 699 541 A1

FIG. 3A

FIG. 3B

1B

EP 4 699 541 A1

FIG. 4

CONTROL UNIT 21

IMAGING CONTROL UNIT 211

DISPLAY CONTROL UNIT 213

CALCULATION UNIT 20 22

BODY MOTION CLASSIFICATION UNIT 221

(FEATURE EXTRACTION UNIT 222)
(IMAGE SELECTION UNIT 223)

DEVICE POSITION CALCULATION UNIT 225

(PARAMETER CALCULATION UNIT 226)
(THREE-DIMENSIONAL POSITION CALCULATION UNIT 227)

# FIG. 5

START

PERFORM IMAGING AT PLURALITY OF IMAGING POSITIONS WITHIN ANGLE RANGE AND COLLECT PLURALITY OF PIECES OF X-RAY IMAGE DATA — S11

CLASSIFY TYPE OF BODY MOTION OF SUBJECT FROM X-RAY IMAGES — S12

DECIDE ON X-RAY IMAGE GROUP TO BE USED FOR PARAMETER CALCULATION BASED ON CLASSIFICATION — S13

CALCULATE PARAMETER SERVING AS INDEX OF DEGREE OF INFLUENCE OF BODY MOTION FOR EACH COMBINATION OF X-RAY IMAGES INCLUDED IN DECIDED-ON X-RAY IMAGE GROUP — S14

CALCULATE THREE-DIMENSIONAL POSITION OF DEVICE BY USING OPTIMAL COMBINATION OF X-RAY IMAGES — S15

END

FIG. 6

EXAMPLE OF ROTATIONAL DRIVING
OF X-RAY IMAGING APPARATUS

EXAMPLES OF IMAGES ACQUIRED BY
ROTATIONAL DRIVING OF X-RAY IMAGING APPARATUS

FIG. 7

# FIG. 8

PERIODIC

(TRAJECTORY OF VICINITY OF DISTAL END 45b)

APERIODIC

(TRAJECTORY OF VICINITY OF DISTAL END 45b)

# FIG. 9

CASE 1

VECTOR SUM

TH

PERIODIC MOTION

TIME (IRRADIATION ANGLE)

CASE 2

VECTOR SUM

TH

PERIODIC MOTION | APERIODIC MOTION | PERIODIC MOTION

TIME (IRRADIATION ANGLE)

CASE 3

VECTOR SUM

TH

PERIODIC MOTION | APERIODIC MOTION

TIME (IRRADIATION ANGLE)

EP 4 699 541 A1

FIG. 10

EP 4 699 541 A1

FIG. 11

FIG. 12

FIG. 13

# FIG. 14

22

CALCULATION UNIT

221

BODY MOTION CLASSIFICATION UNIT

222

FEATURE EXTRACTION UNIT

224

POSITION CORRECTION UNIT

223

IMAGE SELECTION UNIT

226

PARAMETER CALCULATION UNIT

227

THREE-DIMENSIONAL POSITION
CALCULATION UNIT

# FIG. 15

```
┌─────────────────────────────────────┐
│               START                  │
└─────────────────────────────────────┘
                  │
                  ▼
┌─────────────────────────────────────┐
│ PERFORM IMAGING AT PLURALITY OF      │      S11
│ IMAGING POSITIONS WITHIN ANGLE       │
│ RANGE AND COLLECT PLURALITY OF       │
│ PIECES OF X-RAY IMAGE DATA           │
└─────────────────────────────────────┘
                  │
                  ▼
┌─────────────────────────────────────┐
│ DETECT AND CORRECT POSITION OF       │      S11-2
│ FEATURE POINT                        │
└─────────────────────────────────────┘
                  │
                  ▼
┌─────────────────────────────────────┐
│ CLASSIFY TYPE OF BODY MOTION OF      │      S12
│ SUBJECT FROM X-RAY IMAGES            │
└─────────────────────────────────────┘
                  │
                  ▼
┌─────────────────────────────────────┐
│ DECIDE ON X-RAY IMAGE GROUP TO BE    │      S13
│ USED FOR PARAMETER CALCULATION       │
│ BASED ON CLASSIFICATION              │
└─────────────────────────────────────┘
                  │
                  ▼
┌─────────────────────────────────────┐
│ CALCULATE THREE-DIMENSIONAL          │      S14, S15
│ POSITION                             │
└─────────────────────────────────────┘
                  │
                  ▼
┌─────────────────────────────────────┐
│               END                    │
└─────────────────────────────────────┘
```

# FIG. 16

EP 4 699 541 A1

Europäisches
Patentamt

European
Patent Office

Office européen
des brevets

# EUROPEAN SEARCH REPORT

**Application Number**

EP 25 19 1967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/175338 A1 (MATSUZAKI KAZUKI [JP] ET AL) 9 June 2022 (2022-06-09) | 1,3-8,10 | INV.<br>A61B6/12 |
| A | * paragraph [0025] - paragraph [0080]; figures * | 2,9 | A61B6/00 |
| A | US 2020/226779 A1 (MATSUZAKI KAZUKI [JP]) 16 July 2020 (2020-07-16)<br>* paragraph [0042] - paragraph [0086]; figures * | 1-10 | |
| A | US 2019/076103 A1 (MISTRETTA CHARLES A [US] ET AL) 14 March 2019 (2019-03-14)<br>* paragraph [0062]; figures * | 1-10 | |

**TECHNICAL FIELDS
SEARCHED      (IPC)**

A61B
G06T

The present search report has been drawn up for all claims

| Place of search | Date of completion of the search | Examiner |
|---|---|---|
| Munich | 17 December 2025 | Strubel, Christine |

## ANNEX TO THE EUROPEAN SEARCH REPORT
## ON EUROPEAN PATENT APPLICATION NO.

EP 25 19 1967

17-12-2025

| Patent document cited in search report | Publication date | Patent family member(s) | Publication date |
|---|---|---|---|
| US 2022175338 A1 | 09-06-2022 | CN 114617564 A | 14-06-2022 |
| | | JP 7565772 B2 | 11-10-2024 |
| | | JP 2022091427 A | 21-06-2022 |
| | | US 2022175338 A1 | 09-06-2022 |
| US 2020226779 A1 | 16-07-2020 | CN 111093505 A | 01-05-2020 |
| | | JP 6806655 B2 | 06-01-2021 |
| | | JP 2019069037 A | 09-05-2019 |
| | | US 2020226779 A1 | 16-07-2020 |
| | | WO 2019073681 A1 | 18-04-2019 |
| US 2019076103 A1 | 14-03-2019 | TW 201919544 A | 01-06-2019 |
| | | US 2019076102 A1 | 14-03-2019 |
| | | US 2019076103 A1 | 14-03-2019 |
| | | WO 2019055288 A1 | 21-03-2019 |

**EP 4 699 541 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2024140084 A
- JP 5587861 B
- JP 2022091427 A